# FASCICULE DE BREVET EUROPEEN

(11) **EP 4 326 345 B1**
(45) Date de publication et mention de la délivrance du brevet: **31.12.2025**
(21) Numéro de dépôt: 22723712.0
(22) Date de dépôt: 21.04.2022
(51) Int. Cl.: A61L 2/00, A61L 2/08, A61L 2/28, B01F 23/00, B65D 77/06, C12M 1/00, C12M 1/34

(54) **PROCEDE DE STERILISATION D'UN ENSEMBLE COMPRENANT AU MOINS UN DISPOSITIF A USAGE UNIQUE POUR FLUIDE BIOPHARMACEUTIQUE**
VERFAHREN ZUR STERILISIERUNG EINER ANORDNUNG MIT MINDESTENS EINER EINWEGVORRICHTUNG FÜR BIOPHARMAZEUTISCHE FLÜSSIGKEIT
METHOD FOR STERILIZING AN ASSEMBLY COMPRISING AT LEAST ONE SINGLE-USE DEVICE FOR BIOPHARMACEUTICAL FLUID

(30) Priorité: 23.04.2021 FR 2104255
(43) Date de publication de la demande: 28.02.2024
(73) Titulaire: Sartorius Stedim FMT, 13400 Aubagne (FR)
(72) Inventeur: DOREY, Samuel, 83330 LE BEAUSSET (FR); MENIER, Marie-Christine, 13600 LA CIOTAT (FR); NASCIMENTO-BROOKS, Lara, HITCHIN SG4 0QY (GB)
(74) Mandataire: Novagraaf International SA
(86) Numéro de dépôt international: PCT/FR2022/000036
(87) Numéro de publication internationale: WO 2022/223885

(56) Documents cités:
- WO-A1-2017/021653
- DE-A1- 102019 109 210
- US-A1- 2004 033 160
- US-A1- 2005 053 194
- US-A1- 2013 139 618
- US-A1- 2019 329 197

## Description

### Domaine technique

La présente divulgation relève du domaine de la stérilisation des dispositifs à usage unique destinés à recevoir du fluide biopharmaceutique, et plus particulièrement, de la stérilisation par rayons X de palettes ou nacelles contenant un ou plusieurs conteneurs à usage unique destinés à recevoir du fluide biopharmaceutique, c'est-à-dire un produit issu de la biotechnologie (milieux de cultures, cultures cellulaires, solutions tampon, liquides de nutrition artificielle, produits sanguins et dérivés de produits sanguins) ou un produit pharmaceutique ou plus généralement un produit destiné à être utilisé dans le domaine médical.

### Technique antérieure

Une poche (ou conteneur) de type bioréacteur biopharmaceutique est un dispositif qui prend en charge un environnement biologiquement actif. Il peut être composé d'une enveloppe de film, polycarbonate, polyéthylène, ou polypropylène et comprend une cuve destinée à recevoir les produits biopharmaceutiques ou biologiques.

Le bioréacteur peut contenir différents capteurs, comme des capteurs de température ou de pressions, pour suivre l'évolution de l'environnement biologiquement actif. La taille du bioréacteur est variable et peut aller communément de 50 à 2000L. Un exemple d'un tel récipient est décrit dans le brevet US2013/139618, qui présente un conteneur biopharmaceutique comprenant une ou plusieurs entrées et sorties, au moins un dispositif de mélange et au moins un capteur. Ce capteur mesure la conductivité, l'impédance, la capacitance et la température du contenu biopharmaceutique. Un autre exemple est décrit dans le brevet WO2017/021653.

Avant commercialisation, le bioréacteur est traditionnellement irradié au moyen de rayons gamma afin de détruire des micro-organismes qui pourraient être présents et qui pourraient venir corrompre le fonctionnement du bioréacteur par la suite. Le rayonnement gamma est un rayonnement électromagnétique émis lors de la désintégration d'une source radioactive, le cobalt 60. L'énergie du rayonnement émis permet de détruire les microorganismes et peut permettre, à certaines doses, de stériliser un produit. Pour stériliser les produits, on les achemine automatiquement au sein d'une cellule d'irradiation. Les produits sont souvent déjà emballés, et superposés sur un plateau d'une palette ou d'une nacelle pour former un ensemble de produits qui permet de les transporter en lots. L'ensemble reçoit alors une dose contrôlée de rayons gamma, avant d'être acheminée hors de la cellule d'irradiation. L'ensemble peut subir plusieurs irradiations, par exemple, selon plusieurs faces, en faisant tourner l'ensemble sur elle-même, pour s'assurer que toutes les parties de l'ensemble ont reçu l'irradiation. L'énergie absorbée au cours du rayonnement est mesurée en kilogray (kGy). Cette énergie absorbée, mesurée à l'aide d'un dosimètre, dépend de plusieurs facteurs, notamment la durée d'exposition, l'intensité du rayonnement, la densité du matériau et la taille de l'emballage.

Le brevet US2005/053194 décrit un procédé et un dispositif d'irradiation par rayons X de palettes installées sur table tournante. La répartition de la dose absorbée dans le colis est mesurée expérimentalement au moyen d'un réseau de dosimètres en triacétate de cellulose (CTA) et de dosimètres radiochromiques disposés selon une matrice tridimensionnelle à l'intérieur du conditionnement.

Si le rayonnement gamma est à une dose trop élevée, il peut détériorer voire détruire certains composants du bioréacteur, comme par exemple les capteurs. De ce fait, à l'heure actuelle, le traitement par rayons gamma ne permet pas à proprement dit une stérilisation des bioréacteurs. En effet, la relative fragilité des capteurs qui se trouvent dans ces conteneurs ne permet pas de l'irradier à une dose qui permettrait la stérilisation, mais se cantonne à une dose en dessous de celle-ci. On parle alors simplement de « conteneur irradié ». Cette irradiation permet certes une stérilité relative (c'est-à-dire environ 1 organisme sur 100 000 survit), mais elle ne permet de remplir la norme ISO11137 de stérilité en milieu de santé (c'est-à-dire environ 1 organisme sur 1 million survit).

### Résumé

La présente divulgation vient améliorer la situation.

Il est proposé un procédé de stérilisation par rayons X d'un ensemble comprenant un support et un dispositif à usage unique destiné à recevoir un fluide biopharmaceutique, la densité de l'ensemble étant hétérogène, l'ensemble ayant une hauteur de 65% ou plus d'une taille d'une fenêtre de rayonnement par rayons X, la hauteur étant selon une direction normale au support, le dispositif à usage unique comprenant un capteur et un dispositif de mélange, le procédé comprenant : - la pose d'une pluralité de dosimètres répartie sur et/ou dans le dispositif à usage unique suivant une disposition prédéfinie, un dosimètre étant disposé sur le capteur et/ou sur un port du capteur, et un autre dosimètre étant disposé sur le dispositif de mélange, - la formation de l'ensemble suivant une configuration prédéfinie par disposition du dispositif à usage unique équipé de la pluralité de dosimètres dans un emballage sur le support, - la répétition d'un passage de l'ensemble devant la fenêtre de rayonnement par rayons X, selon une première face puis selon une deuxième face de l'ensemble, à plusieurs couples puissance-temps d'irradiation, par incrémentation de couple puissance-temps entre un couple puissance-temps minimal et un couple puissance-temps maximal, suivie d'une cartographie de la dose d'irradiation reçue par chacun de la pluralité des dosimètres pour chacun des couples puissance-temps incrémentés, la première face et la deuxième face contenant la hauteur de l'ensemble, la deuxième face étant opposée à la première face ; et - la détermination d'un couple puissance-temps de radiation par rayons X optimal pour lequel la cartographie révèle qu'après le passage selon la première face et selon la deuxième face tous les dosimètres enregistrent une dose d'irradiation au-dessus d'une dose minimale et que le dosimètre associé au capteur enregistre une dose d'irradiation en dessous d'une dose maximale, la dose d'irradiation minimale étant définie comme la dose à partir de laquelle la stérilité est effective, et la dose d'irradiation maximale étant définie comme étant la dose à partir de laquelle l'irradiation par rayons X détériore le capteur.

Dans le cas d'un conteneur par exemple, du fait de la présence d'éléments de matériaux différents (par exemple un arbre du dispositif de mélange est en polytéréphtalate d'éthylène alors qu'une paroi du conteneur est en polypropylène, ou encore des roulements à billes qui peuvent contenir des éléments en céramique et/ou en métal), la densité du conteneur, et par conséquent de l'ensemble est hétérogène. Ainsi, pour un rayonnement donné, la dose de radiation reçue peut varier d'un point à l'autre du conteneur. Un matériau plus dense recevra une dose d'irradiation moindre qu'un matériau moins dense. En conséquence de quoi, la stérilité peut ne pas être assurée partout si une dose insuffisante est reçue dans les zones de l'ensemble de forte densité. Un matériel est dit d'avoir une forte densité lorsque sa densité est supérieure à 0,6 g/cm³. D'autre part, le conteneur comporte des éléments fragiles, tel que le capteur qui peuvent être détériorés si une dose d'irradiation trop importante est appliquée. Le procédé tient donc en compte ces paramètres pour permettre à un ensemble comprenant un (ou plusieurs emballages) comprenant chacun un (ou plusieurs) conteneur d'être stérilisée en un minimum de passages. Ceci est d'autant plus pertinent lorsque l'ensemble est de hauteur de 65% ou plus d'une taille d'une fenêtre de rayonnement par rayons X. Selon un mode de réalisation, l'ensemble est de hauteur de 65% ou plus d'une taille d'une fenêtre de rayonnement par rayons X. Selon un mode de réalisation, l'ensemble est de hauteur de 70% ou plus d'une taille d'une fenêtre de rayonnement par rayons X. Selon un mode de réalisation, l'ensemble est de hauteur de 75% ou plus d'une taille d'une fenêtre de rayonnement par rayons X. Selon un mode de réalisation, l'ensemble est de hauteur de 80% ou plus d'une taille d'une fenêtre de rayonnement par rayons X. Selon un mode de réalisation, l'ensemble est de hauteur de 85% ou plus d'une taille d'une fenêtre de rayonnement par rayons X. Selon un mode de réalisation, l'ensemble est de hauteur de 90% ou plus d'une taille d'une fenêtre de rayonnement par rayons X. Dans ce cas, la fenêtre de rayonnement peut créer des effets de bord, c'est-à-dire des zones en périphérie de la fenêtre de rayonnement qui ne reçoivent pas l'irradiation prescrite. Le procédé présenté ici permet aussi de s'affranchir au moins en partie de ces effets de bord.

Les caractéristiques exposées dans les paragraphes suivants peuvent, optionnellement, être mises en œuvre. Elles peuvent être mises en œuvre indépendamment les unes des autres ou en combinaison les unes avec les autres :
- le procédé comprend de plus une étape préalable de détermination de la dose maximale et/ou de détermination de la dose minimale.
- le dispositif à usage unique contient une pluralité d'éléments sensibles, les éléments sensibles étant définis comme des éléments qui se détériorent au-delà d'une dose d'irradiation prédéterminée, le capteur faisant partie de la pluralité d'éléments sensibles, et dans lequel l'étape de pose des dosimètres comprend la pose d'un dosimètre sur chacun de la pluralité d'éléments sensibles, et dans l'étape de détermination du couple puissance-temps de radiation par rayons X optimal, le couple puissance-temps optimal est le couple pour lequel la cartographie révèle que tous les dosimètres enregistrent une dose d'irradiation au-dessus de la dose minimale et que les dosimètres associés éléments sensibles enregistrent une dose d'irradiation en dessous de la dose maximale, et la dose maximale étant définie comme étant la dose la plus basse à partir de laquelle l'irradiation par rayons X détruit la fonctionnalité d'un des éléments sensibles.
- avant chaque passage de l'ensemble devant la fenêtre de rayonnement à un couple puissance-temps d'irradiation donné, une nouvelle pluralité de dosimètres encore non irradiés est posée sur un nouveau dispositif à usage unique non encore irradié à la disposition prédéterminée pour former un nouvel ensemble non encore irradié.
- avant chaque passage de l'ensemble selon la deuxième face devant la fenêtre de rayonnement à un couple puissance-temps d'irradiation donné, une nouvelle pluralité de dosimètres encore non irradiés est posée sur un nouveau dispositif à usage unique non encore irradié à la disposition prédéterminée pour former un nouvel ensemble non encore irradié
- la dose minimale d'irradiation est définie par la norme ISO11137.
- la dose d'irradiation minimale est d'au moins 25kGy, préférentiellement d'au moins 27,5kGy et encore plus préférentiellement d'au moins 30kGy.
- la dose d'irradiation maximale est comprise entre 45 et 50kGy, préférentiellement 47kGy.
- les couples puissance-temps sont des couples pris parmi des combinaisons de puissance d'irradiation et de temps d'irradiation compris entre une puissance minimale de 75 kW et une puissance maximale de 1100 kW, et un temps d'irradiation minimal de 0,1h et un temps d'irradiation maximal de 20h.
- l'incrémentation en temps de l'irradiation se fait par tranches comprises entre 0,1h et 2h.
- l'incrémentation en puissance de l'irradiation se fait par tranches comprises entre 50 et 200 kW.
- le procédé comprend de plus la répétition d'un passage de l'ensemble devant la fenêtre de rayonnement selon deux autres faces opposées de l'ensemble.
- l'emballage comprend plusieurs dispositifs à usage unique et/ou l'ensemble comprend plusieurs emballages disposés sur le support.
- le procédé comprend de plus l'irradiation au couple puissance-temps optimal d'un nouvel ensemble comprenant un nouvel emballage et un nouveau dispositif à usage unique de formes et tailles identiques à celle des étapes précédentes, le dispositif à usage unique et emballage étant disposés dans la configuration prédéterminée, l'irradiation se faisant selon la première face puis la deuxième face de l'ensemble.

Selon un autre aspect, il est proposé un ensemble comprenant : - un support sur lequel repose un emballage contenant un dispositif à usage unique à usage unique et destiné à recevoir un fluide biopharmaceutique, la densité de l'ensemble étant hétérogène, l'ensemble ayant une hauteur de 65% ou plus d'une taille d'une fenêtre de rayonnement par rayons X, le dispositif à usage unique comprenant un capteur et un dispositif de mélange, l'ensemble étant stérilisée suivant le procédé ci-dessus.

### Brève description des dessins

D'autres caractéristiques, détails et avantages apparaîtront à la lecture de la description détaillée ci-après, et à l'analyse des dessins annexés, sur lesquels :
**Fig. 1**

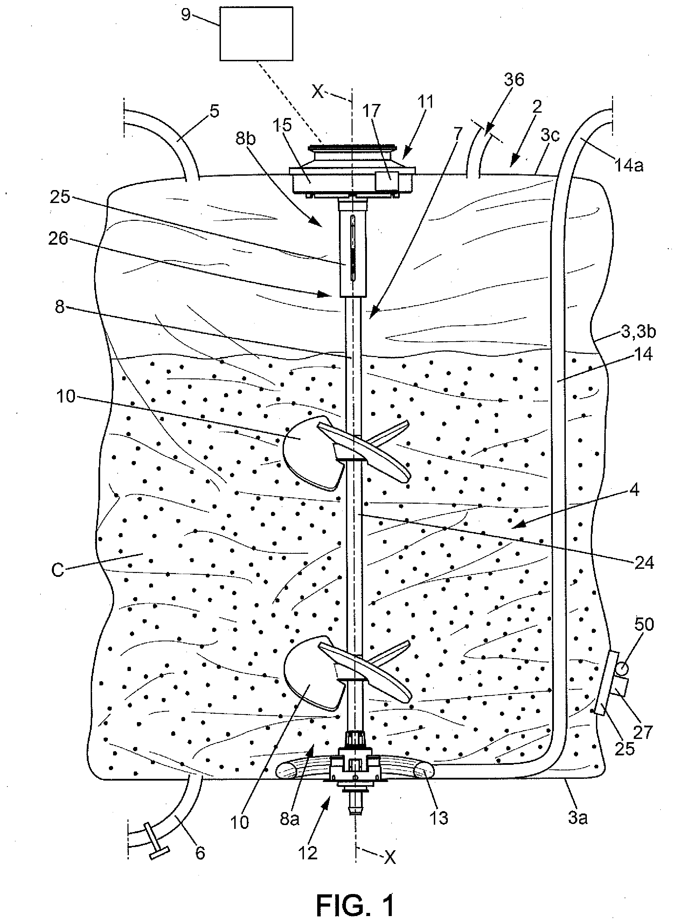

   [Fig. 1] est une vue schématique d'un dispositif à usage unique destiné à recevoir un fluide biopharmaceutique, selon un mode de réalisation.
**Fig. 2**

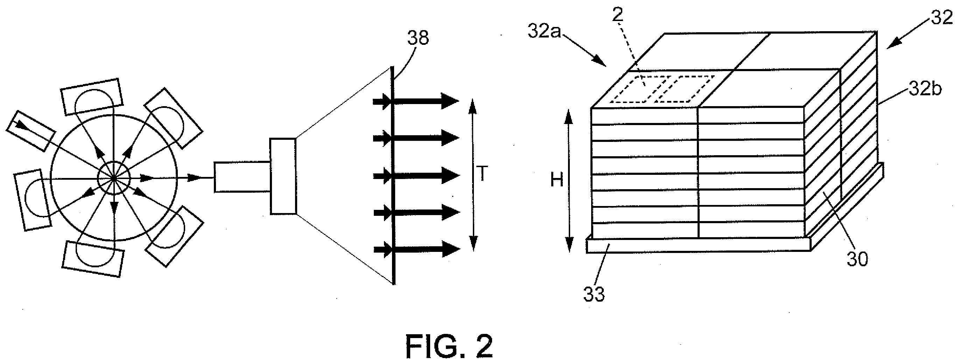

   [Fig. 2] est une vue schématique d'un ensemble contenant plusieurs emballages, chaque emballage contenant un ou plusieurs dispositifs à usage unique, l'ensemble étant disposée devant une fenêtre de rayonnement par rayons X, selon un mode de réalisation.
**Fig. 3**

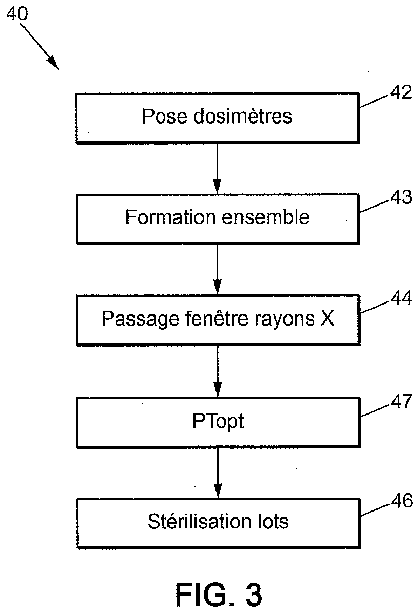

   [Fig. 3] est un ordinogramme d'un procédé de stérilisation d'un ensemble comprenant au moins un dispositif à usage unique destiné à recevoir un fluide biopharmaceutique, selon un mode de réalisation.

### Description des modes de réalisation

Il est maintenant fait référence à la **figure 1****,** qui montre une vue schématique d'un exemple de **dispositif à usage unique 2** à usage unique recevant un **fluide biopharmaceutique C.** Le dispositif à usage unique peut être une poche ou un conteneur, ou bien un filtre. Lorsque la poche ou conteneur 2 est destiné(e) à une réaction chimique, le conteneur 2 peut alors être appelé « bioréacteur ».

Le dispositif à usage unique 2 peut être destiné à recevoir le fluide biopharmaceutique C pour des opérations de transport, de culture cellulaire, de mélange et/ou réactions chimiques, et/ou filtration. Le fluide biopharmaceutique peut être un produit issu de la biotechnologie - milieux de cultures, cultures cellulaires, solutions tampon, liquides de nutrition artificielle, produits sanguins et dérivés de produits sanguins - ou un produit pharmaceutique ou plus généralement un produit destiné à être utilisé dans le domaine médical.

Le dispositif à usage unique 2 est selon un mode de réalisation un conteneur 2 formé par une **paroi 3,** préférentiellement en matière plastique, par exemple en polycarbonate, polyéthylène, ou polypropylène. La paroi 3 est flexible et étanche au fluide biopharmaceutique C. La paroi 3 du conteneur 2 peut comprendre une **partie inférieure 3a,** une **partie latérale 3b** et une **partie supérieure 3c,** par exemple formée par un ou plusieurs tronçons solidarisés, soudés, les uns avec les autres. Le conteneur 2 délimite ainsi un **espace intérieur 4,** apte à recevoir une certaine quantité du fluide biopharmaceutique C. La paroi 3 peut être totalement ou partiellement transparente ou translucide afin de pouvoir visualiser depuis l'extérieur le fluide biopharmaceutique C dans l'espace intérieur 4.

Le conteneur 2 est à usage unique, et peut avoir une capacité comprise entre 10 et 5 000 litres, en fonction des besoins et des applications. Selon un autre mode de réalisation, la capacité du conteneur 2 est 10, 50, 100, 200, 1000, 2000 ou 5000 litres.

Le conteneur 2 présente un **axe principal XX** selon lequel il s'étend. Le conteneur 2 peut être fait de sorte que l'espace intérieur 4 soit de forme plus ou moins cylindrique, rectangulaire ou même plutôt plat, notamment pour les poches de petits volumes.

Le conteneur 2 peut, en fonction de sa taille être plus ou moins complexe et contenir plus ou moins de composants. Au minimum le conteneur 2 comprend un **capteur 27** et un **dispositif de mélange 7** du fluide biopharmaceutique C. Le conteneur 2 peut comprendre plusieurs capteur 27 (par exemple des capteurs par contact, des capteurs optiques, capteurs de température, capteurs de pression) et/ou plusieurs dispositifs de mélange 7. Le capteur 27 et le dispositif de mélange 7 seront décrits plus en détails ci-dessous.

Le conteneur 2 peut comporter un ou plusieurs **ports 5** traversant **d'introduction** du fluide biopharmaceutique C ou de composants du fluide biopharmaceutique C, coopérant avec un ou plusieurs orifices d'introduction ménagés dans le conteneur 2. Le conteneur 2 peut comporter également au moins un **port 6** traversant **de vidange** du fluide biopharmaceutique C, coopérant avec au moins un orifice de vidange ménagé dans le conteneur 2. Le port de vidange 6 est apte à être obturé à chaque fois que nécessaire et au contraire ouvert pour la vidange. On entend par « port », un moyen de connexion ou de liaison physique. Un tel port est traversant lorsqu'il s'agit d'assurer une fonction de mise en communication entre l'espace intérieur 4 et l'extérieur du conteneur 2, par exemple pour l'introduction ou la vidange de ce qui doit être placé ou est placé dans le conteneur 2. Un tel port peut également être non traversant lorsqu'il s'agit d'assurer une fonction de maintien d'un organe du récipient-mélangeur. Des conduits, poches, réservoirs, le cas échéant souples, peuvent être associés au port d'introduction, en communication fluidique et avec une connexion étanche et le cas échéant amovible. De même, des conduits, poches, réservoirs, le cas échéant souples peuvent être associés au port de vidange 6, en communication fluidique et avec une connexion étanche et le cas échéant amovible. Dans la réalisation représentée sur la figure 1, la plus basse du conteneur 2. Cependant, cette réalisation n'est pas limitative et un ou plusieurs ports d'introduction peuvent être situés dans la partie inférieure 3a ou dans la partie latérale 3b du conteneur 2. Le conteneur 2 peut également comporter un **dispositif d'aération 13** apte à délivrer au fluide biopharmaceutique C une certaine quantité de gaz d'aération. Ce dispositif 13 permet ainsi l'aération 10 de ce qui se trouve dans l'espace intérieur 4 du conteneur 2, qu'il s'agisse du fluide biopharmaceutique C, ou d'une partie de ses composants. Le dispositif d'aération 13 peut comprendre un **dispositif d'amenée de gaz d'aération 14** ayant au moins un **élément tubulaire 14a** s'étendant avec communication fluidique depuis l'extérieur du conteneur 2. Au dispositif d'aération 13 peut être associé fonctionnellement au moins un **port d'évacuation de gaz d'aération 36** ménagé dans la partie supérieure 3c de la paroi 3 du conteneur 2. Un tel port d'évacuation de gaz d'aération 36 permet d'évacuer du conteneur 2, vers l'extérieur, le gaz qui n'a pas été mélangé avec le fluide biopharmaceutique C du conteneur 2. Le conteneur 2 peut, dans certaines réalisations, comporter également d'autres ports connus en soi, par exemple de montage d'un moyen fonctionnel, apte à assurer le maintien d'un organe tel que typiquement la collecte ou la mesure de données, ou la prise d'échantillon aux fins d'analyse.

Le dispositif de mélange 7 permet le mélange de ce qui se trouve dans l'espace intérieur 4 du conteneur 2, qu'il s'agisse du fluide biopharmaceutique C, ou d'une partie de ses composants. Le dispositif de mélange 7 comprend au moins un **arbre 8,** apte à être entraîné, notamment magnétiquement, en rotation par un **moteur 9** et à entraîner en rotation au moins un **organe de mélange 10** montés sur l'arbre 8. Le ou les organes de mélange 10 sont substantiellement espacés de la partie inférieure 3a et de la partie latérale 3b de la paroi 3 du conteneur 2. L'organe de mélange 10 peut se présenter sous la forme d'une hélice ayant un moyeu supportant plusieurs pales. L'arbre 8 peut être de longueur fixe ou avoir une longueur ajustable. L'arbre 8 peut être mis en rotation par le moteur 9 (visible partiellement à la figure 2), extérieur au conteneur 2.

Le conteneur 2 comprend également au moins un **premier palier 11,** adjacent à la partie supérieure 3c de la paroi 3, avec lequel coopère la partie supérieure 8b de l'arbre 8. Le premier palier 11 comporte une collerette rigide. On entend ici par « collerette », une pièce rigide en forme générale de paroi pleine, au moins sensiblement plate, placée à plat, et destinée au maintien. Cette collerette est fixée de façon rigide et étanche à la partie supérieure 3c de la paroi 3 du conteneur 2. Plus précisément, la collerette est formée d'une matière sensiblement rigide, de préférence une matière plastique rigide, en forme de paroi ou de plaquette raccordée au conteneur 2, au centre de la 40 partie supérieure 3c. Cette collerette peut être raccordée à la paroi 3 du conteneur 2 de toute manière appropriée de façon à former un joint rigide et hermétique entre les matières respectives, rigide et flexible de la collerette et de la paroi 3.

Selon un mode de réalisation, l'arbre 8 du dispositif de mélange 7 est situé tout entier dans l'espace intérieur 4. Ainsi, l'arbre 8 s'étend de façon rectiligne entre une **extrémité inférieure 8a** et une **extrémité supérieure 8b.** Lorsque le conteneur 2 est dans une position apte à son fonctionnement, l'arbre 8 s'étend de façon verticale suivant l'axe principal XX, l'extrémité inférieure 8a étant située vers la partie inférieure 3a du conteneur 2 tandis que l'extrémité supérieure 8b est située vers la partie supérieure 3c de conteneur 2.

Selon un mode de réalisation, le moteur 9 d'entraînement, permet l'entrainement magnétique en rotation de l'arbre 8. À cet effet, le moteur 9 comprend un disque rotatif menant situé à l'extérieur du conteneur 2. L'arbre 8 comprend alors un **disque rotatif mené 15** destiné à coopérer fonctionnellement, notamment magnétiquement, avec le disque rotatif menant 30 du moteur 9. Plus particulièrement, le disque rotatif mené 15 comprend une pluralité **d'aimants 17,** qui sont intégrés par tout moyen de fixation ou de construction, afin de permettre la rotation de l'arbre 8 lors de la rotation du disque rotatif menant du moteur 9. Le disque rotatif mené 15 est solidaire, notamment en rotation, de l'arbre 8.

Le conteneur 2 comprend, selon un mode de réalisation, une fixation, ou **port 25** pour fixer le capteur 27 à la paroi 3. Selon un mode de réalisation, le capteur 27 est une sonde par contact avec le fluide biopharmaceutique C, et la fixation 25 est une pièce annulaire qui entoure un port de mesure, et protège le capteur 27. Ce port de mesure peut être distinct du port 5 et du port de vidange 6. Le capteur 27 peut être une sonde par contact qui permet de mesurer des paramètres relatifs au fluide biopharmaceutique C qui se trouvera sans l'espace intérieur 4 du conteneur 2, comme la pression, le pH, la température, la colorimétrie, de biomasse ou la conductimétrie. D'autres type de capteurs sont envisagés pour le conteneur 2, comme par exemple, des capteurs optiques, capteurs de température, et/ou des capteurs de pression, à la place ou en addition du capteur dans le port de mesure 24. Certains capteurs peuvent être associés à des zones de fixation qui n'entourent pas un port ou une ouverture du conteneur 2. Ils peuvent alors être mis simplement sur la paroi du conteneur 2 (intérieur ou extérieur), via une fixation ou porte capteur 25.

Le conteneur 2 peut avoir des éléments additionnels selon le type d'application.

Le dispositif à usage unique 2 peut être mis sous emballage de façon à être transporté facilement et de façon sécuritaire de l'usine de fabrication vers sa destination finale, et notamment pendant sa stérilisation. Pour ce, et en référence à la **figure 2****,** le dispositif à usage unique 2 peut être placé (optionnellement avec d'autres) dans un **emballage 30** sur un **support 33** formant ainsi **un ensemble 32** contenant un ou plusieurs emballages empilés. Dans l'exemple de la figure 2, de manière illustrative, l'emballage 30 contient quatre dispositifs à usage unique 2. L'emballage 30 pourrait contenir moins de quatre ou plus de quatre dispositifs à usage unique 2. Par exemple, l'emballage 30 pourrait ne contenir qu'un seul dispositif à usage unique 2. Ce serait par exemple le cas pour un conteneur 2 de grande taille, par exemple 500 L ou plus. Le dispositif à usage unique 2 pourrait aussi être plié, avec optionnellement l'arbre 8 rétracté, de façon à prendre une empreinte au sol moindre dans l'emballage 30. Les dispositifs à usage unique 2 à l'intérieur d'un même emballage 30 (dans le cas où l'emballage 30 contiendrait plusieurs dispositifs à usage unique 2) peuvent avoir tous une même orientation, ou bien avoir une orientation différente. Les dispositifs à usage unique 2 pourraient, selon un mode de réalisation, être disposés tête bêche, afin de rendre l'emballage 30 plus compact per exemple.

Si la taille des emballages le permet, plusieurs emballages 30 peuvent être disposés sur le support 33 de façon superposée et/ou adjacents les uns aux autres. La répartition par empilement ou en disposition adjacente des emballages 30 peut être choisie suivant le type et/ou la taille du ou des dispositifs à usage unique 2. Dans l'exemple de la figure 2, l'ensemble 32 contient quatre piles d'emballages 30, chaque pile contenant huit emballages 30.

Le support 33 peut aussi avoir plusieurs formes et tailles. Selon l'exemple de la figure 2, le support33 est une base rectangulaire plane, de type plateau ou palette. Selon un mode de réalisation, le support 33 a une dimension de 80 cm par 120 cm. Selon un autre mode de réalisation, le support 33 a une dimension de 100 cm par 120 cm. Le support 33 peut être fait de bois et avoir des encoches latérales permettant de la transporter. Un chariot élévateur peut ainsi être utilisé pour déplacer le support 33. Le support 33 peut aussi être mis sur un tapis roulant.

Le support 33 montré sur les figures et de forme palette. Il se pourrait cependant que le support 33 fasse partie d'une nacelle (aussi parfois appelée balancelle). Selon un mode de réalisation, la nacelle comprend deux plateaux disposés verticalement l'un par rapport à l'autre, chaque plateau étant un support pour des emballages de conteneurs. La nacelle permet ainsi de transporter deux fois plus de produits qu'une palette simple. La nacelle est typiquement accrochée par le haut, ce qui permet de la faire avancer (via un convoyeur) et de la faire tourner sur elle-même, selon les besoins.

Comme les dispositifs à usage unique 2 sont destinés à recevoir un fluide biopharmaceutique, il est préférable de s'assurer que les dispositifs à usage unique 2 soient stériles. En effet, la présence de micro-organismes indésirables dans l'espace intérieur 4 pourrait provoquer une réaction ou contamination du fluide biopharmaceutique C. Il est alors coutume de réaliser la stérilité lorsque le dispositif à usage unique 2 est déjà emballé et mis sur le support 33. La stérilisation peut se faire par rayons X. Pour ce, l'ensemble 32 se trouvant sur un convoyeur peut être acheminée vers une fenêtre de rayonnement afin de recevoir une dose d'irradiation qui permettrait d'éliminer les micro-organismes.

Toujours en référence à la **figure 2** et en complément à la **figure 3****,** un **procédé 40** de stérilisation utilise les rayons X afin de stériliser les dispositifs à usage unique 2 en lots via la stérilisation de l'ensemble 32 comprenant le support 33 sur lequel repose au moins un emballage 30 comprenant au moins un dispositif à usage unique, tel que le conteneur 2. Le dispositif à usage unique pourrait être une poche ou un filtre. Pour l'illustration du procédé 40, il sera fait référence au cas où le dispositif à usage unique est un conteneur. Le conteneur 2 est à usage unique et destiné à recevoir le fluide biopharmaceutique C. Il comprend au minimum le capteur 27 et le dispositif de mélange 7. L'ensemble 32 peut être présentée devant une **fenêtre de rayonnement par rayons X 38** pour stériliser une pluralité de conteneurs 2 en lots.

Le procédé 40 est particulièrement adapté dans les cas où la densité du conteneur 2 (et par conséquent de l'ensemble 32) est hétérogène. Le procédé 40 permet de déterminer une irradiation par rayons X permettant la stérilisation, et que cette stérilisation soit effective dans les zones de forte densité du conteneur 2 telles que le dispositif de mélange 7, sans pour autant endommager les éléments sensibles du conteneur 2, tels que capteur 27. Un matériel est dit d'avoir une forte densité lorsque sa densité est supérieure à 0,6 g/cm³.

Le procédé 40 est aussi particulièrement adapté dans les cas où l'ensemble 32 a une **hauteur H** qui se rapproche de celle de la fenêtre 38 de rayonnement par rayons X. En effet, pour procéder à l'irradiation, l'ensemble 32 est présentée devant la fenêtre de rayonnement 38. On choisit d'irradier une **face latérale 32a** de l'ensemble 32 qui contient la hauteur H de l'ensemble 32, la hauteur H étant définie comme étant dans une direction normale au support 33. On considère que la hauteur H de l'ensemble 32 se rapproche de celle de la fenêtre 38 de rayonnement par rayons X lorsque la hauteur H de l'ensemble est de 65% ou plus d'une **taille T** de la fenêtre 38 de rayonnement par rayons X. En effet, lorsque la taille du produit à irradier est substantiellement celle de la fenêtre 38 de rayonnement, le rayonnement peut créer des effets de bord, c'est-à-dire des zones en périphérie de la fenêtre de rayonnement 38 qui ne reçoivent pas l'irradiation prescrite. La méthode présentée ici permet de s'affranchir au moins en partie de ces effets de bord. Selon un mode de réalisation, l'ensemble est de hauteur de 65% ou plus d'une taille d'une fenêtre de rayonnement par rayons X. Selon un mode de réalisation, l'ensemble est de hauteur de 70% ou plus d'une taille d'une fenêtre de rayonnement par rayons X. Selon un mode de réalisation, l'ensemble est de hauteur de 75% ou plus d'une taille d'une fenêtre de rayonnement par rayons X. Selon un mode de réalisation, l'ensemble est de hauteur de 80% ou plus d'une taille d'une fenêtre de rayonnement par rayons X. Selon un mode de réalisation, l'ensemble est de hauteur de 85% ou plus d'une taille d'une fenêtre de rayonnement par rayons X. Selon un mode de réalisation, l'ensemble est de hauteur de 90% ou plus d'une taille d'une fenêtre de rayonnement par rayons X.

Le procédé 40 commence à **l'étape 42** par la pose d'une pluralité (au moins deux) de **dosimètres 50** répartie sur et/ou dans le conteneur 2. Certains dosimètres peuvent aussi être disposés sur l'emballage 30 et/ou le support 33. Les dosimètres 50 permettent de déterminer une dose effective d'irradiation reçue. Cette donnée permet de s'assurer d'une part que la dose reçue est au-dessus d'une dose requise pour la stérilisation du conteneur 2, et d'autre part que la dose ne dépasse pas une valeur à partir de laquelle un composant du conteneur 2 pourrait être détérioré.

La dose reçue, pour une irradiation donnée, est dépendante de l'hétérogénéité du conteneur 2. En effet pour un rayonnement donné, la densité d'un matériau influe sur la dose d'irradiation reçue : plus un matériau est dense plus la dose reçue est faible. De ce fait, au moins un dosimètre 50 est disposé sur le dispositif de mélange 7. Le dispositif de mélange 7 fait partie des zones du conteneur de plus forte densité (relativement aux autres parties du conteneur 2). De manière préférentielle plusieurs dosimètres sont répartis dans les zones de plus forte densité, autre que le dispositif de mélange 7, afin de contrôler la dose d'irradiation effectivement reçue en différents endroits du conteneur 2. Selon un mode de réalisation, une zone de plus forte densité est une zone pour laquelle la densité localement est supérieure à la densité moyenne du conteneur 2. Selon un mode de réalisation, une zone de plus forte densité est une zone pour laquelle la densité localement est deux fois supérieure à la densité moyenne du conteneur 2. Les zones de forte densité peuvent être déterminées en fonction du matériau et/ou de l'épaisseur du matériau en considération. Par exemple, les éléments suivants peuvent être considérés comme des zones de forte densité par rapport au reste du conteneur 2, et notamment de la paroi flexible 3 : l'arbre de mélange supportant les hélices, les paliers haut et bas comportant des roulements à billes.

La densité de l'emballage 30 et du support 33 peut être assimilée à la densité du conteneur 2 dans la détermination des zones de plus forte densité. En effet, selon un mode de réalisation, l'emballage 30 est fait de carton, et le support 33 de bois ou d'acier, les deux étant de construction substantiellement homogène. De ce fait, ils ne représentent pas une densité additionnelle significative par rapport au conteneur 2 non emballé. Par conséquent, une détermination des zones de forte densité (à des fins de dépôt des dosimètres 50 pour l'étape 42) au niveau du conteneur 2 sera considérée comme une détermination des zones de forte densité au niveau de l'ensemble 32 en général.

D'autre part, un dosimètre 50 est disposé sur le capteur 27, afin de s'assurer que le capteur 27 ne reçoit pas une dose d'irradiation qui l'endommagerait. Le dosimètre 50 peut être disposé sur le capteur 27 ou en proche proximité du capteur 27, comme par exemple sur le port du capteur 25. Plus qu'un dosimètre 50 peut être disposé au niveau du capteur 27.

Selon un mode de réalisation, des dosimètres 50 sont répartis dans les zones fragiles du conteneur 2 autres que le capteur 27, déterminées comme pouvant être altérées si exposés à une dose trop importante de rayons X. En effet, le capteur 27 peut ne pas être le seul type d'élément du conteneur 2 pouvant être altéré par une dose d'irradiation trop importante. Les matériaux formant la paroi 3 du conteneur 2 subissent des modifications chimiques, présentent une oxydation des polymères et la génération d'espèces oxydées à l'intérieur et à la surface des polymères, suite à une exposition trop forte aux rayons X. Cette génération d'espèces oxydées peut entraîner la génération de radicaux libres et donc, une modification des polymères. Ces radicaux libres peuvent induire une agrégation et une oxydation des protéines des produits dans les bioconteneurs à usage unique. De ce fait, selon un mode de réalisation, des dosimètres 50 sont aussi disposés sur la paroi 3.

Selon un mode de réalisation, plusieurs dosimètres 50 sont disposés sur le ou les capteurs 27, et plusieurs dosimètres sont disposés sur le ou les dispositifs de mélange 7. Selon un mode de réalisation, les dosimètres 50 sont répartis uniformément sur et/ou dans le conteneur 2. Selon un autre mode de réalisation, les dosimètres 50 sont répartis de façon non-uniforme sur le conteneur 2, de façon à privilégier les zones de forte densité et les éléments fragiles du conteneur 2.

On peut choisir autant de dosimètres que de précision souhaitée dans le relevé des mesures sur le volume du conteneur 2. Le nombre et la répartition des dosimètres dans et/ou sur le conteneur 2 influe sur une cartographie de l'irradiation reçue.

Il existe des dosimètres de type I ou de type II. Les dosimètres de type I peuvent utiliser la solution de Fricke, la solution de bichromate avec évaluation spectrophotométrique, la solution cérique-céreux avec spectrophotométrie ou potentiométrie, ou la solution éthanol-chlorobenzène avec analyse de titrage pour déterminer la dose d'irradiation absorbée pendant le rayonnement. Les dosimètres de type Il comprennent des colorimètres de procédé, du triacétate de cellulose, une matrice polymère contenant du fluorure de lithium (photofluorescent), des systèmes Perspex et des films et liquides radiochromiques.

Afin de poser les dosimètres sur et/ou dans le conteneur 2, le conteneur 2 est sacrifié, en ce qu'il est tailladé pour permettre l'installation des dosimètres 50, et ne pourra pas être utilisé ultérieurement selon son usage habituel.

De l'étape 42, on passe à **l'étape 43,** où l'ensemble 32 est formée suivant une configuration prédéfinie par la disposition du conteneur 2 équipé de la pluralité de dosimètres 50 dans l'emballage 30 puis sur le support 33. Selon un mode de réalisation, si l'ensemble 32 contient plusieurs emballages 30 qui chacun contient un ou plusieurs conteneurs 2, chaque conteneur 2 est équipé des dosimètres 50 et est placé dans l'emballage 30 et sur l'ensemble 32 de la même façon qu'il va l'être lors de la stérilisation en lots (étape 47) (c'est-à-dire suivant la configuration prédéfinie), afin de recréer les conditions de passage des palettes 32 équipées des emballages de conteneurs 2 devant la fenêtre de rayonnement 38. Il est porté aussi attention à l'orientation de l'ensemble 32 vis-à-vis de la fenêtre de rayonnement 38 qui fait partie de la configuration prédéfinie, et à reproduire lors de l'étape 47 de stérilisation en lots.

De l'étape 43, on passe à l**'étape 44** qui consiste en la répétition d'un passage de l'ensemble 32 contenant le conteneur 2 équipé de la pluralité de dosimètres 50 devant la fenêtre 38 de rayonnement par rayons X, selon une **première face 32a** de l'ensemble 32 puis suivant une **deuxième face 32b,** à différents couples puissance-temps d'irradiation par rayons X. La répétition de passages se fait par incrémentation de couple puissance-temps entre un couple **puissance-temps minimal (PTmin)** et un couple **puissance-temps maximal (PTmax).** À chaque irradiation, la dose reçue par chaque dosimètre est enregistrée pour un couple puissance-temps donné et pour chaque face 32a,32b donnée. Selon un mode de réalisation, l'ensemble 32 passe successivement devant la fenêtre 38 de rayonnement par rayons X suivant la première face 32a puis suivant la deuxième face 32b à un couple puissance-temps donné, avant de passer successivement devant la fenêtre 38 de rayonnement par rayons X suivant la première face 32a puis suivant la deuxième face 32b à un autre couple puissance temps-donné, et ainsi de suite. La première face 32a et la deuxième face 32b sont des surfaces latérales opposées de l'ensemble 32. Elles contiennent la hauteur H de l'ensemble 32. La fenêtre 38 de rayonnement par rayons X est donc disposée latéralement à l'ensemble 32 afin d'irradier une surface latérale de l'ensemble 32. Si une face du dessus de l'ensemble 32 avait été préférée pour effectuer l'irradiation, la fenêtre de rayonnement 38 aurait été disposée au-dessus de l'ensemble 32.

Afin de permettre l'irradiation selon la première face latérale 32a puis selon la deuxième face latérale opposée 32b, l'ensemble 32 est retourné avant de repasser devant la fenêtre 38 de rayonnement par rayons X. Le retournement correspond à une rotation à 180 degrés selon une direction normale au support 33. La rotation peut se faire de plusieurs façons. Le support 33 avec les emballages 30 et conteneurs 2 peut être soulevé et tourné de 180 degrés. Si l'ensemble est une nacelle, la nacelle peut être retournée par rotation sur elle-même autour d'un pivot vertical par laquelle elle est suspendue. Si la nacelle comprend deux supports disposés verticalement l'un par rapport à l'autre, chaque support contenant des emballages de conteneurs, de sorte que la hauteur de palette-nacelle soit plus grande que celle de la fenêtre de rayonnement 38, la position de la nacelle peut aussi être ajustée verticalement par rapport à la taille T de la fenêtre de rayonnement 38 afin d'irradier chacun des supports de la nacelle.

Selon un mode de réalisation, les couples puissance-temps sont des couples pris parmi des combinaisons de puissance d'irradiation et de temps d'irradiation compris entre une puissance minimale de 75 kW et une puissance maximale de 1100 kW, et un temps d'irradiation minimal de 0,1h et un temps d'irradiation maximal de 20h. Selon un mode de réalisation, l'incrémentation en temps d'irradiation se fait par tranches comprises entre de 0,1h et 2h. Selon un mode de réalisation, lequel l'incrémentation en puissance d'irradiation se fait par tranches comprises entre de 50 et 200 kW. L'incrémentation peut être constant ou variable entre chaque couple puissance-temps.

Comme les doses d'irradiation sont cumulatives, on peut irradier de façon successive le même ensemble 32 et soustraire entre chaque mesure pour déterminer la dose reçue pour un couple puissance-temps donné.

D'une autre façon, on peut changer l'ensemble 32 à chaque mesure, de sorte à ce que les dosimètres 50 ne reçoivent qu'une dose d'irradiation. À cette fin, une pluralité d'ensembles 32 contenant les dosimètres 50 est préparée à l'étape 42, avec les dosimètres 50 positionnées de façon identique dans chaque ensemble 32, et les conteneurs 2 et emballages 30 étant dans la configuration prédéfinie de l'ensemble 32. L'utilisation de la pluralité d'ensembles 32 contenant les dosimètres 50 pour un passage unique devant la fenêtre de rayonnement peut être préférée du fait que les dosimètres sont généralement étalonnés entre 0-~80/100 kGy, limitant une exposition prolongée (>100 kGy).

L'incrémentation de couple puissance-temps entre un **couple puissance-temps minimal (PTmin)** et un **couple puissance-temps maximal (PTmax)** peut se faire en faisant d'abord varier les puissances, puis les temps, ou bien en faisant d'abord varier les temps, puis ensuite les puissances ou encore en faisant varier les deux. Les couples puissance-temps minimal et maximal sont trouvés de façon expérimentale.

La dose reçue par chaque dosimètre pour une irradiation à un couple puissance-temps est enregistrée de façon à créer une cartographie de la dose d'irradiation reçue par chacun de la pluralité des dosimètres en fonction de leur position sur et/ou dans le conteneur (et possiblement sur l'emballage 30 et/ou sur le support 33) pour chacun des couples puissance-temps incrémentés. Si le même ensemble 32 contenant les dosimètres 50 est soumis plus d'une fois à un couple puissance-temps d'irradiation, on procède par soustraction afin de déterminer réellement quelle est la dose reçue pour chaque couple puissance-temps.

La cartographie pour chaque couple puissance-temps d'irradiation peut être enregistrée dans une mémoire pour traitement à l'étape suivante.

De l'étape 44 on passe à **l'étape 46,** qui consiste en la détermination d'un couple **puissance-temps de radiation par rayons X optimal PTopt.** Le couple optimal est choisi parmi les couples dont la cartographie a été établie à l'étape 44. Le couple puissance-temps optimal PTopt est défini comme étant un couple puissance-temps pour lequel la cartographie révèle qu'après le passage selon la première face 32a et selon la deuxième face 32b tous les dosimètres 50 enregistrent une dose d'irradiation au-dessus d'une **dose minimale Dmin** et que le dosimètre associé au capteur 27 (et de façon générale aux éléments sensibles du conteneur 2) enregistre une dose d'irradiation en dessous d'une **dose maximale Dmax.** Les éléments sensibles sont les éléments du dispositif à usage unique 2 qui se détériorent au-delà d'une dose d'irradiation prédéterminée. Par exemple, dans le cas du conteneur 2, parmi les éléments sensibles se trouvent les capteurs, les éléments électroniques et certains éléments mécaniques. Par exemple, si le capteur 27 est à lecture optique à travers une membrane, la couleur de la membrane peut être détériorée par une irradiation trop importante, ce qui va fausser la lecture du capteur. Un même problème peut se poser pour les électrodes des capteurs de biomasse, ou des sondes pH sèches. La dose d'irradiation minimale Dmin est définie comme la dose à partir de laquelle la stérilité est effective. Selon un mode de réalisation, la dose d'irradiation minimale est définie par la norme ISO11137. Selon un mode de réalisation, la dose d'irradiation minimale est d'au moins 25kGy, préférentiellement d'au moins 27,5kGy et encore plus préférentiellement d'au moins 30kGy. La dose d'irradiation maximale Dmax est définie comme étant la dose à partir de laquelle l'irradiation par rayons X détruit la fonctionnalité d'un capteur 27 (et plus généralement des éléments sensibles). Selon un mode de réalisation, la dose d'irradiation maximale Dmax est entre 45 et 50kGy. Selon un mode de réalisation, la dose d'irradiation maximale Dmax est de 47kGy. Si le conteneur 2 comprend plusieurs éléments sensibles, chacun ayant une dose palier associée à partir de laquelle il se détériore, on prendra de façon préférentielle une dose d'irradiation maximale Dmax commune à tous, comme étant la plus basse des doses paliers des éléments sensibles.

Une fois le couple puissance-temps optimal établi, on passe à **l'étape 47** qui consiste en la stérilisation en lots de plusieurs ensembles 32 acheminés préférentiellement de façon automatique devant la fenêtre de rayonnement 38 par un convoyeur. L'irradiation se fait au couple puissance-temps optimal PTopt déterminé à l'étape 46 sur un nouvel ensemble 32 comprenant un nouvel emballage 30 et un nouveau conteneur 2 de formes et tailles identiques à celle des étapes précédentes (sauf pour la présence de dosimètres qui sont absents à l'étape 47). Le conteneur 2 et emballage 30 sont disposés dans la configuration prédéterminée. L'irradiation se fait selon la première face 32a puis la deuxième face 32b de l'ensemble 32 au couple puissance-temps optimal PTopt avant de passer à un autre ensemble à irradier. Selon un autre mode de réalisation, l'irradiation se fait au couple puissance-temps optimal PTopt selon la première face 32a pour chaque ensemble 32 à irradier, puis les ensembles repassent devant la fenêtre de rayonnement 38 mais cette fois ci selon la deuxième face 32b pour être irradié au couple puissance-temps optimal PTopt.

## Revendications

1. **Procédé (40)** de stérilisation par rayons X d'un **ensemble (32)** comprenant un **support (33)** et un **dispositif** à usage unique **(2)** destiné à recevoir un **fluide biopharmaceutique (C),** la densité de l'ensemble (32) étant hétérogène, l'ensemble (32) ayant une hauteur (H) de 65% ou plus d'**une taille (T)** d'une **fenêtre (38)** de rayonnement par rayons X, la hauteur (H) étant selon une direction normale au support (33), le dispositif à usage unique (2) comprenant un **capteur (27)** et un **dispositif de mélange (7),** le procédé (40) comprenant :
- la pose d'une pluralité de **dosimètres (50)** répartie sur et/ou dans le dispositif à usage unique (2) suivant une disposition prédéfinie, un dosimètre (50) étant disposé sur le capteur (27) et/ou sur un **port (25)** du capteur (27), et un autre dosimètre (50) étant disposé sur le dispositif de mélange (7),
- la formation de l'ensemble (32) suivant une configuration prédéfinie par disposition du dispositif à usage unique (2) équipé de la pluralité de dosimètres (50) dans un emballage (30) sur le support (33)
- la répétition d'un passage de l'ensemble (32) devant la fenêtre de rayonnement (38) par rayons X, selon une **première face (32a)** puis selon une **deuxième face (32b)** de l'ensemble (32), à plusieurs couples **puissance-temps d'irradiation (PTi),** par incrémentation de couple puissance-temps entre un couple **puissance-temps minimal (PTmin)** et un couple **puissance-temps maximal (PTmax),** suivie d'une cartographie de la dose d'irradiation reçue par chacun de la pluralité des dosimètres (50)
pour chacun des couples puissance-temps incrémentés, la première face (32a) et la deuxième face (32b) contenant la hauteur (H) de l'ensemble (32), la deuxième face (32b) étant opposée à la première face (32a) ; et
- la détermination d'un **couple puissance-temps** de radiation par rayons X **optimal (PTopt)** pour lequel la cartographie révèle qu'après le passage selon la première face (32a) et selon la deuxième face (32b) tous les dosimètres (50) enregistrent une dose d'irradiation au-dessus d'une **dose minimale (Dmin)** et que le dosimètre **(50)** associé au capteur (27) enregistre une dose d'irradiation er dessous d'une **dose maximale (Dmax),** la dose d'irradiation minimale (Dmin) étant définie comme la dose à partir de laquelle la stérilité est effective, et la dose d'irradiation maximale (Dmax) étant définie comme étant la dose à partir de laquelle l'irradiation par rayons X détériore le capteur (27).

2. Procédé (40) selon la revendication 1, comprenant de plus une étape préalable de détermination de la dose maximale (Dmax) et/ou de détermination de la dose minimale (Dmin).

3. Procédé (40) selon la revendication 1 ou 2, dans lequel le dispositif à usage unique (2) contient une pluralité d'éléments sensibles, les éléments sensibles étant définis comme des éléments qui se détériorent au-delà d'une dose d'irradiation prédéterminée, le capteur (27) faisant partie de la pluralité d'éléments sensibles, et dans lequel l'étape de pose des dosimètres (50) comprend la pose d'un dosimètre (50) sur chacun de la pluralité d'éléments sensibles, et dans l'étape de détermination du couple puissance-temps de radiation par rayons X optimal (PTopt), le couple puissance-temps optimal est le couple pour lequel la cartographie révèle que tous les dosimètres (50) enregistrent une dose d'irradiation au-dessus de la dose minimale (Dmin) et que les dosimètres (50) associés aux éléments sensibles enregistrent une dose d'irradiation en dessous de la dose maximale (Dmax), et la dose maximale (Dmax) étant définie comme étant la dose la plus basse à partir de laquelle l'irradiation par rayons X détruit la fonctionnalité d'un des éléments sensibles.

4. Procédé (40) selon l'une des revendications précédentes, dans lequel la dose minimale d'irradiation (Dmin) est définie par la norme ISO11137.

5. Procédé (40) selon l'une des revendications précédentes, dans lequel la dose d'irradiation minimale (Dmin) est d'au moins 25kGy, préférentiellement d'au moins 27,5kGy et encore plus préférentiellement d'au moins 30kGy.

6. Procédé (40) selon l'une des revendications précédentes, dans lequel la dose d'irradiation maximale (Dmax) est comprise entre 45 et 50kGy, préférentiellement 47kGy.

7. Procédé (40) selon l'une des revendications précédentes, dans lequel les couples puissance-temps sont des couples pris parmi des combinaisons de puissance d'irradiation et de temps d'irradiation compris entre une puissance minimale de 75 kW et une puissance maximale de 1100 kW, et un temps d'irradiation minimal de 0,1h et un temps d'irradiation maximal de 20h.

8. Procédé (40) selon l'une des revendications précédentes, dans lequel l'incrémentation en temps de l'irradiation se fait par tranches comprises entre 0,1h et 2h.

9. Procédé (40) selon l'une des revendications précédentes, dans lequel l'incrémentation en puissance de l'irradiation se fait par tranches comprises entre 50 et 200 kW.

10. Procédé (40) selon l'une des revendications précédentes, comprenant de plus la répétition d'un passage de l'ensemble (32) devant la fenêtre (38) de rayonnement selon deux autres faces opposées de l'ensemble (32).

11. Procédé (40) selon l'une des revendications précédentes, dans lequel l'emballage (30) comprend plusieurs dispositifs à usage unique (2) et/ou l'ensemble (32) comprend plusieurs emballages (30) disposés sur le support (33).

12. Procédé (40) selon l'une des revendications précédentes, dans lequel avant chaque passage de l'ensemble (32) devant la fenêtre de rayonnement (38) à un couple puissance-temps d'irradiation (PTi) donné, une nouvelle pluralité de dosimètres (50) encore non irradiée est posée sur un nouveau dispositif à usage unique (2) non encore irradié à la disposition prédéterminée pour former un nouvel ensemble non encore irradié, le nouveau dispositif à usage unique (2) étant de formes et tailles identiques à celle des étapes précédentes.

13. Procédé (40) selon la revendication précédente, comprenant de plus l'irradiation au couple puissance-temps optimal (PTopt) du nouvel ensemble (32), l'irradiation se faisant selon une première face (32a) puis une deuxième face (32b) du nouvel l'ensemble (32).

14. **Ensemble (32)** comprenant :
- un **support (33)** sur lequel repose un **emballage (30)** contenant un **dispositif à usage unique (2)** et destiné à recevoir un **fluide biopharmaceutique (C),** la densité de l'ensemble (32) étant hétérogène, l'ensemble (32) ayant une hauteur (H) de 65% ou plus d'une **taille (T)** d'une **fenêtre (38)** de rayonnement par rayons X, le dispositif à usage unique (2) comprenant un **capteur (27)** et un **dispositif de mélange (7),**
l'ensemble (32) étant stérilisé suivant le procédé de l'une des revendications 1 à 11.

## Patentansprüche

1. Verfahren (40) zur Sterilisierung mittels Röntgenstrahlen einer Anordnung (32), die einen Träger (33) und eine Einwegvorrichtung (2) umfasst, die zur Aufnahme eines biopharmazeutischen Fluids (C) bestimmt ist, wobei die Dichte der Anordnung (32) heterogen ist, die Anordnung (32) eine Höhe (H) von 65 % oder mehr einer Größe (T) eines Röntgenstrahlen-Bestrahlungsfensters (38) hat, wobei die Höhe (H) in einer Richtung senkrecht zum Träger (33) verläuft, wobei die Einwegvorrichtung (2) einen Sensor (27) und eine Mischvorrichtung (7) umfasst, wobei das Verfahren (40) umfasst:
- das Anbringen einer Vielzahl von Dosimetern (50), die an und/oder in der Einwegvorrichtung (2) in einer vordefinierten Anordnung verteilt sind, wobei ein Dosimeter (50) am Sensor (27) und/oder an einem Port (25) des Sensors (27) angeordnet ist, und ein weiteres Dosimeter (50) an der Mischvorrichtung (7) angeordnet ist,
- das Bilden der Anordnung (32) gemäß einer vordefinierten Konfiguration durch Anordnen der mit der Vielzahl von Dosimetern (50) ausgestatteten Einwegvorrichtung (2) in einer Verpackung (30) auf dem Träger (33),
- das Wiederholen eines Vorbeiziehens der Anordnung (32) vor dem Röntgenstrahlen-Bestrahlungsfenster (38) gemäß einer ersten Seite (32a) und anschließend gemäß einer zweiten Seite (32b) der Anordnung (32) anhand mehrerer Bestrahlungs-Leistungs-Zeit-Paare (PTi) mittels Leistungs-Zeit-Paar-Inkrementierung zwischen einem minimalen Leistungs-Zeit-Paar (PTmin) und einem maximalen Leistungs-Zeit-Paar (PTmax), gefolgt von einer Kartierung der von jedem der Vielzahl von Dosimetern (50) empfangenen Strahlendosis für jedes der inkrementierten Leistungs-Zeit-Paare, wobei die erste Seite (32a) und die zweite Seite (32b) die Höhe (H) der Anordnung (32) enthalten, wobei die zweite Seite (32b) der ersten Seite (32a) gegenüberliegt; und
- das Bestimmen eines optimalen Röntgenstrahlenbestrahlungs-Leistungs-Zeit-Paares (PTopt), für das die Kartierung zeigt, dass nach dem Vorbeiziehen gemäß der ersten Seite (32a) und gemäß der zweiten Seite (32b) alle Dosimeter (50) eine Strahlendosis über einer Mindestdosis (Dmin) registrieren und dass das dem Sensor (27) zugeordnete Dosimeter (50) eine Strahlendosis unter einer maximalen Dosis (Dmax) registriert, wobei die minimale Strahlendosis (Dmin) als die Dosis definiert ist, ab der die Sterilität wirksam ist, und die maximale Strahlendosis (Dmax) als die Dosis definiert ist, ab der die Bestrahlung mit Röntgenstrahlen den Sensor (27) beschädigt.

2. Verfahren (40) nach Anspruch 1, das zusätzlich einen vorangehenden Schritt des Bestimmens der maximalen Dosis (Dmax) und/oder des Bestimmens der minimalen Dosis (Dmin) umfasst.

3. Verfahren (40) nach Anspruch 1 oder 2, wobei die Einwegvorrichtung (2) eine Vielzahl empfindlicher Elemente enthält, wobei die empfindlichen Elemente als Elemente definiert sind, die sich über eine vorbestimmte Strahlendosis hinaus verschlechtern, wobei der Sensor (27) Teil der Vielzahl empfindlicher Elemente ist, und wobei der Schritt des Anbringens der Dosimeter (50) das Anbringen eines Dosimeters (50) an jedem von der Vielzahl empfindlicher Elemente umfasst, und wobei in dem Schritt des Bestimmens des optimalen Röntgenstrahlenbestrahlungs-Leistungs-Zeit-Paares (PTopt) das optimale Leistungs-Zeit-Paar das Paar ist, für das die Kartierung zeigt, dass alle Dosimeter (50) eine Strahlendosis über der minimalen Dosis (Dmin) registrieren und dass die den empfindlichen Elementen zugeordneten Dosimeter (50) eine Strahlendosis unterhalb der maximalen Dosis (Dmax) registrieren, und wobei die maximale Dosis (Dmax) als die niedrigste Dosis definiert ist, ab der die Bestrahlung durch Röntgenstrahlen die Funktionsfähigkeit eines der empfindlichen Elemente zerstört.

4. Verfahren (40) nach einem der vorstehenden Ansprüche, wobei die minimale Strahlendosis (Dmin) von der Norm ISO 11137 bestimmt wird.

5. Verfahren (40) nach einem der vorstehenden Ansprüche, wobei die minimale Strahlendosis (Dmin) mindestens 25 kGy, vorzugsweise mindestens 27,5 kGy und noch bevorzugter mindestens 30 kGy beträgt.

6. Verfahren (40) nach einem der vorstehenden Ansprüche, wobei die maximale Strahlendosis (Dmax) zwischen 45 und 50 kGy, vorzugsweise 47 kGy, liegt.

7. Verfahren (40) nach einem der vorstehenden Ansprüche, wobei die Leistungs-Zeit-Paare Paare sind, die aus Kombinationen von Bestrahlungsleistung und Bestrahlungszeit zwischen einer minimalen Leistung von 75 kW und einer maximalen Leistung von 1100 kW und einer minimalen Bestrahlungszeit von 0,1 h und einer maximalen Bestrahlungszeit von 20 h ausgewählt sind.

8. Verfahren (40) nach einem der vorstehenden Ansprüche, wobei die Bestrahlungszeit-Inkrementierung in Schritten zwischen 0,1 h und 2 h erfolgt.

9. Verfahren (40) nach einem der vorstehenden Ansprüche, wobei die Bestrahlungsleistungs-Inkrementierung in Schritten zwischen 50 und 200 kW erfolgt.

10. Verfahren (40) nach einem der vorstehenden Ansprüche, das zusätzlich die Wiederholung eines Vorbeiziehens der Anordnung (32) vor dem Bestrahlungsfenster (38) gemäß zwei weiteren gegenüberliegenden Seiten der Anordnung (32) umfasst.

11. Verfahren (40) nach einem der vorstehenden Ansprüche, wobei die Verpackung (30) mehrere Einwegvorrichtungen (2) umfasst und/oder die Anordnung (32) mehrere auf dem Träger (33) angeordnete Verpackungen (30) umfasst.

12. Verfahren (40) nach einem der vorstehenden Ansprüche, wobei vor jedem Vorbeiziehen der Anordnung (32) vor dem Bestrahlungsfenster (38) anhand eines gegebenen BestrahlungsLeistungs-Zeit-Paares (PTi) eine neue Vielzahl von noch nicht bestrahlten Dosimetern (50) auf einer neuen, noch nicht bestrahlten Einwegvorrichtung (2) mit der vorbestimmten Anordnung angebracht wird, um eine neue, noch nicht bestrahlte Anordnung zu bilden, wobei die neue Einwegvorrichtung (2) von identischen Formen und Größen wie die der vorhergehenden Schritte ist.

13. Verfahren (40) nach vorstehendem Anspruch, das zusätzlich die Bestrahlung der neuen Anordnung (32) mit dem optimalen Leistungs-Zeit-Paar (PTopt) umfasst, wobei die Bestrahlung gemäß einer ersten Seite (32a), dann einer zweiten Seite (32b) der neuen Anordnung (32) erfolgt.

14. Anordnung (32), umfassend:
- einen Träger (33), auf dem eine Verpackung (30) ruht, die eine Einwegvorrichtung (2) enthält und zur Aufnahme eines biopharmazeutischen Fluids (C) bestimmt ist, wobei die Dichte der Anordnung (32) heterogen ist, die Anordnung (32) eine Höhe (H) von 65 % oder mehr einer Größe (T) eines Röntgenstrahlen-Bestrahlungsfensters (38) hat, wobei die Einwegvorrichtung (2) einen Sensor (27) und eine Mischvorrichtung (7) umfasst,
wobei die Anordnung (32) gemäß dem Verfahren nach einem der Ansprüche 1 bis 11 sterilisiert wird.

## Claims

1. A method (40) for sterilizing by X-rays an assembly (32) comprising a support (33) and a single-use device (2) for receiving a biopharmaceutical fluid (C), the density of the assembly (32) being heterogeneous, the assembly (32) having a height (H) of 65% or more of a size (T) of an X-ray irradiation window (38), the height (H) being considered according to a direction normal to the support (33), the single-use device (2) comprising a sensor (27) and a mixing device (7), the method (40) comprising:
- placing a plurality of dosimeters (50) distributed on and/or in the single-use device (2) according to a predefined arrangement, one dosimeter (50) being arranged on the sensor (27) and/or on a port (25) of the sensor (27), and another dosimeter (50) being arranged on the mixing device (7),
- forming the assembly (32) according to a predefined configuration by arranging the single-use device (2) equipped with the plurality of dosimeters (50) in a package (30) on the support (33);
- repeating a passage of the assembly (32) in front of the X-ray irradiation window (38), according to a first face (32a) then according to a second face (32b) of the assembly (32), at several irradiation power-time pairs (PTi), while incrementing the power-time pair between a minimum power-time pair (PTmin) and a maximum power-time pair (PTmax), followed by a mapping of the irradiation dose received by each of the plurality of dosimeters (50) for each of the incremented power-time pairs, the first face (32a) and the second face (32b) containing the height (H) of the assembly (32), the second face (32b) being opposite to the first face (32a); and
- determining an optimum X-ray irradiation power-time pair (PTopt) for which the mapping reveals that after passage according to the first face (32a) and according to the second face (32b) all dosimeters (50) record an irradiation dose above a minimum dose (Dmin) and that the dosimeter (50) associated with the sensor (27) records an irradiation dose below a maximum dose (Dmax), the minimum irradiation dose (Dmin) being defined as the dose from which sterility is effective, and the maximum irradiation dose (Dmax) being defined as the dose from which X-ray irradiation deteriorates the sensor (27).

2. The method (40) according to claim 1, further comprising a prior step of determining the maximum dose (Dmax) and/or determining the minimum dose (Dmin).

3. The method (40) according to claim 1 or 2, wherein the single-use device (2) contains a plurality of sensitive elements, the sensitive elements being defined as elements that deteriorate beyond a predetermined irradiation dose, the sensor (27) forming part of the plurality of sensitive elements, and wherein the step of placing the dosimeter (50) comprises placing a dosimeter (50) on each of the plurality of sensitive elements, and in the step of determining the optimum X-ray radiation power-time pair (PTopt), the optimum power-time pair is the pair for which mapping reveals that all dosimeters (50) record an irradiation dose above the minimum dose (Dmin) and that the dosimeters (50) associated with sensitive elements record an irradiation dose below the maximum dose (Dmax), and the maximum dose (Dmax) being defined as the lowest dose from which X-ray irradiation destroys the functionality of one of the sensitive elements.

4. The method (40) according to any one of the preceding claims, wherein the minimum irradiation dose (Dmin) is defined by standard ISO11137.

5. The method (40) according to any one of the preceding claims, wherein the minimum irradiation dose (Dmin) is at least 25kGy, preferably at least 27.5kGy and still more preferably at least 30kGy.

6. The method (40) according to any one of the preceding claims, wherein the maximum irradiation dose (Dmax) is comprised between 45 and 50kGy, preferably 47kGy.

7. The method (40) according to any one of the preceding claims, wherein the power-time pairs are pairs taken from among combinations of radiation power and irradiation time comprised between a minimum power of 75 kW and a maximum power of 1100 kW, and a minimum irradiation time of 0.1 h and a maximum irradiation time of 20 h.

8. The method (40) according to any one of the preceding claims, wherein the time increment of the radiation is done in increments comprised between 0.1 h and 2 h.

9. The method (40) according to any one of the preceding claims, wherein the power increment of the irradiation is done in increments comprised between 50 and 200 kW.

10. The method (40) according to any one of the preceding claims, further comprising the repetition of a passage of the assembly (32) in front of the radiation window (38) according to two other opposite faces of the assembly (32).

11. The method (40) according to any one of the preceding claims, wherein the package (30) comprises several single-use devices (2) and/or the assembly (32) comprises several packages (30) arranged on the support (33).

12. The method (40) according to any one of the preceding claims, wherein before each passage of the assembly (32) in front of the radiation window (38) at a given irradiation power-time pair (PTi), a new plurality of dosimeters (50) which have not yet been irradiated is placed on a new single-use device (2) which has not yet been irradiated with the predetermined arrangement to form a new assembly which has not yet been irradiated, the new single-use device (2) being of shapes and sizes identical to those of the previous steps.

13. The method (40) according to the preceding claim, further comprising irradiating at the optimum power-time pair (PTopt) the new assembly (32), the irradiation taking place according to a first face (32a) then a second face (32b) of the new assembly (32).

14. An assembly (32) comprising:
- a support (33) on which a package (30) rests containing a single-use device (2) and intended to receive a biopharmaceutical fluid (C), the density of the assembly (32) being heterogeneous, the assembly (32) having a height (H) of 65% or more of a size (T) of an X-ray irradiation window (38), the single-use device (2) comprising a sensor (27) and a mixing device (7),
the assembly (32) being sterilized according to the method of any one of claims 1 to 11.
